# EUROPÄISCHE PATENTSCHRIFT

(11) **EP 0 432 253 B1**
(45) Veröffentlichungstag und Bekanntmachung des Hinweises auf die Patenterteilung: **02.10.1996**
(21) Anmeldenummer: 90910669.2
(22) Anmeldetag: 04.07.1990
(51) Int. Cl.: A61B 17/58, A61B 17/72

(54) **MARKNAGEL**
MEDULLARY NAIL
CLOU MEDULLAIRE

(30) Priorität: 04.07.1989 DE 3921972
(43) Veröffentlichungstag der Anmeldung: 19.06.1991
(73) Patentinhaber: Baumgart, Rainer, Dipl.-Ing. Dr. med., D-81479 München (DE); BETZ, Augustin, D-82319 Starnberg (DE)
(72) Erfinder: Baumgart, Rainer, Dipl.-Ing. Dr. med., D-81479 München (DE); BETZ, Augustin, D-82319 Starnberg (DE)
(74) Vertreter: Finck, Dieter, Dr.Ing.
(86) Internationale Anmeldenummer: EP9001076
(87) Internationale Veröffentlichungsnummer: WO9100065

(56) Entgegenhaltungen:
- DE-A- 2 224 214
- DE-A- 3 541 597
- DE-B- 2 213 283
- DE-U- 8 907 561
- FR-A- 2 267 080
- FR-A- 2 338 685

## Beschreibung

Die Erfindung betrifft einen Marknagel.

Bekanntlich verwendet man die Marknagelung dazu, im Bereich von langen Röhrenknochen nach Knochenbrüchen die beiden Frakturenden im Sinne einer inneren Schienung gegeneinander ruhigzustellen. Dabei wird nach Aufbohrung des Markraums der Marknagel meist vom proximalen Ende des Röhrenknochens eingeschlagen und nach endgültiger Positionierung mittels mehrerer Querbolzen, die durch die Knochenkortikalis und Fixierungslöcher im Marknagel hindurchgehen, gegen axiale Verschiebung und Verdrehung verriegelt. Da die den Knochen außen umgebende Knochenhaut bei dieser Behandlungsart nicht wesentlich beschädigt wird, ist die für den Heilungsprozeß wichtige Ernährung begünstigt und eine Infektvorbeugung gegeben.

Im fortgeschrittenen Stadium der Knochenbruchheilung kann man die Verriegelungsbolzen auf einer Seite des Frakturspaltes entfernen. Eine axiale Belastung führt nun zu einem Gleiten des entriegelten Knochenanteils und somit zu einer axialen Kompression am Frakturspalt, wodurch sich der neugebildete Knochen verfestigt.

Verlängerungen von Röhrenknochen oder Defektüberbrückungen nach Trümmerbrüchen, einer Knochenentzündung oder nach Entfernen von Tumoren im Bereich langer Röhrenknochen werden bisher meist mit Hilfe von äußeren Fixierungssystemen durchgeführt. Die Knochenenden werden dabei mit Knochenschrauben oder Drähten gefaßt, die durch die Haut nach außen mit einem Rahmen verbunden sind. Durch Verschiebung der den jeweiligen Knochenenden zugeordneten Rahmenteile mit Hilfe von Zugschrauben oder Drahtwinden, wird eine Relativbewegung zwischen den Knochenenden zwangsweise erreicht, wobei die geringen Verschiebungsdistanzen fortlaufend durch sich neubildendes Knochengewebe überbrückt werden. Sowohl zur Verlängerung als auch für eine Segmentverschiebung ist zuvor eine Osteotomie erforderlich, wobei bei der Segmentverschiebung das abgetrennte Knochensegment in die Defektzone gezogen wird.

Die bekannten äußeren Fixierungssysteme haben einen ungünstigen Kraftansatzpunkt, so daß Verkantungen auftreten, erfordern aufwendige, den Patienten behindernde Aufbauten, bilden eine ständige Infektionsgefahr durch Eindringen von Keimen über die Knochenschrauben oder die Drähte und ergeben unschöne Narben.

Aus der DE-35 41 597 A1 (Fig. 5) ist zur Streckung eines Röhrenknochens, also zur Reposition nach Fraktur oder zur Korrektur nach Umstellungsosteotomie ein Marknagel bekannt, der eine einen Hohlraum bildende Wand mit einem sich verjüngenden distalen Ende und einem proximalen Eintreibende hat.

In dem Hohlraum ist ein Innenteil längsverschiebbar angeordnet.

In dem Hohlraum ist ferner am Eintreibende ein Einsatz vorgesehen, der eine zentrale Bohrung aufweist.

In dieser zentralen Bohrung sitzt eine Stange.

Im Abstand in Längserstreckung der den Hohlraum bildenden Wand sind quer durchgehende Fixierungslöcher vorgesehen, in welche Bolzen zur Verankerung im Knochen geführt sind.

Im Bereich des Eintreibendes erstreckt sich quer durch die den Hohlraum bildende Wand ein Langloch.

Ein quer durch das Innenteil hindurchgehendes Fixierungsloch ist fluchtend zu dem Langloch in der den Hohlraum bildenden Wand ausgerichtet.

Der Einsatz hat einen Außengewindeabschnitt, der in Eingriff mit einem Innengewinde steht, das in den proximalen Bereich der Innenwand eines Hohlzylinders geschnitten ist, der den Hohlraum bildet.

Der Einsatz hat seinerseits ein Innengewinde, in welches ein Außengewinde an der Stange eingreift, die mit der Stirnseite des Innenteils verbunden ist, welche dem Eintreibende zugewandt ist. Die Gewindeverbindung zwischen Einsatz und Innenwand des Hohlraums ist gegenläufig zu der zwischen Einsatz und Stange oder hat eine unterschiedliche Steigung. Durch Drehung des Einsatzes mit einem speziellen Werkzeug läßt sich die Stange und damit das Innenteil in Längsrichtung des Hohlraums bewegen, wodurch bei entsprechender Fixierung der Querbolzen die gewünschte Streckung des Knochens erreicht werden kann.

Aus der DE-22 24 214 A1 ist ein Marknagel bekannt, der eine einen Hohlraum bildende Wand in Form eines hohlzylindrischen Hülsenteils mit einem sich verjüngenden distalen Ende und einem proximalen Eintreibende hat. In dem Hülsenteil sitzt koaxial in Gewindeeingriff ein Innenteil, das von einer Betätigungsspindel gebildet wird, die vom Eintreibende über das Hülsenteil vorsteht. Mit der Betätigungsspindel steht an ihrem distalen Ende ein Dornteil in Gewindeeingriff, das bei entsprechender Drehung der Betätigungsspindel bezüglich des Hülsenteils axial verschiebbar ist. Innerhalb der Betätigungsspindel sitzt eine Spreizzahnspindel, die am Eintreibende aus der Betätigungsspindel vorsteht und bei entsprechender Drehung Spreizzähne aus dem Dornteil ausfährt.

Der Marknagel wird nach Eintreiben in den Markkanal mit einem selbstschneidenden Außengewinde auf der Außenseite des Hülsenteils im Knochen verankert. Anschließend wird der Dornteil ausgefahren und mit Hilfe der Spreizzähne am Ende des Markkanals fixiert. Dadurch läßt sich der Marknagel ohne Verwendung von Querbolzen und Fixierungslöchern im Markkanal festlegen und in seiner Länge durch den Gewindeeingriff zwischen Betätigungsspindel und Dornteil axial zur Erzielung der gewünschten Verlängerung von mittels Osteotomie durchtrennten Röhrenknochen erreichen.

Die FR-2 267 080 beschreibt verschiedene Ausgestaltungen von implantierbaren Antrieben zur Erzielung einer Verlängerung von mittels Osteotomie durchtrennten Gliedmaßenknochen, die pneumatisch, hydraulisch, elektrisch oder elektromagnetisch arbeiten, die jedoch nicht für den Einbau in den Hohlraum eines Marknagels vorgesehen sind.

Die der Erfindung zugrunde liegende Aufgabe besteht darin, einen Marknagel zu schaffen, der unter Aufrechterhaltung seiner Funktion zur Stabilisierung als innere Schiene gleichzeitig eine axiale Verschiebung von getrennten Knochenteilen voneinander weg mit einem einfachen Antrieb über einen längeren Zeitraum kontinuierlich oder in kleinen Schritten nach der Operation ermöglicht.

Diese Aufgabe wird erfindungsgemäß durch einen Marknagel
- mit einer einen Hohlraum bildenden Wand mit einem sich verjüngenden distalen Ende und einem proximalen Eintreibende,
- mit einem in dem Hohlraum längsverschiebbar angeordneten Innenteil, in das sich ausgehend von seiner dem Eintreibende zugewandten Stirnseite eine koaxiale Bohrung mit Innengewinde erstreckt,
- mit einem im Hohlraum am Eintreibende vorgesehenen Einsatz, der eine zentrale Bohrung aufweist,
- mit einer am Eintreibende vorgesehenen, in der zentralen Bohrung gelagerten Stange, die ein Außengewinde hat, das in Eingriff mit dem Innengewinde der Bohrung des Innenteils steht,
- mit im Abstand in Längserstreckung der den Hohlraum bildenden Wand quer durchgehenden Fixierungslöchern
gemäß einer ersten Alternative
- mit einem im Bereich des Eintreibendes quer durch die den Hohlraum bildende Wand gehenden Langloch und
- mit wenigstens einem quer durch das Innenteil hindurchgehenden, fluchtend zu dem Langloch in der den Hohlraum bildenden Wand ausgerichteten Fixierungsloch
und gemäß einer zweiten Alternative
- mit einem im Bereich des Eintreibendes quer durch das Innenteil gehenden Langloch,
- mit wenigstens einem quer durch die den Hohlraum bildende Wand hindurchgehenden, fluchtend zu dem Langloch in dem Innenteil ausgerichteten Fixierungsloch und
- mit einer Trennfuge in der den Hohlraum bildenden Wand, die die Wand so trennt, daß die beiden Teile axial zueinander geführt auf dem Innenteil verschiebbar sind,
gelöst.

Bevorzugt wird ein mechanischer, pneumatischer, hydraulischer, elektrischer oder elektromagnetischer Antrieb für die Drehung der Stange zur Längsverschiebung des Innenteils eingesetzt.

Zweckmäßigerweise wird der Antrieb innerhalb des Hohlraums angeordnet.

Die Drehung der Stange kann durch einen Drehantrieb in Form einer biegsamen Welle erfolgen, die nach einer genügend langen Strecke im Weichteilgewebe durch die Haut nach außen geführt wird, und die an ihrem proximalen Ende für das Drehen mit einem Sechskant versehen ist. Als Drehantrieb kann auch ein geeigneter Elektrogetriebemotor eingesetzt werden, der an dem proximalen Ende des Marknagel angeflanscht ist oder sogar in dem Lumen des Marknagels untergebracht werden kann. Die Energieversorgung kann mit Hilfe von Batterien als Einheit mit dem Elektromotor oder separat durch Einpflanzung eines Batteriegehäuses in das subkutane Fettgewebe erfolgen, wobei zum Anschluß eine flexible Litze verwendet wird. Die Aktivierung des Elektromotors kann durch Auflage eines Magneten mit Hilfe eines Reed-Kontaktes oder im kontinuierlichen Betrieb erfolgen.

Nach Aufbohren des Markraums und Anlegen einer Osteotomie an einer geeigneten Stelle mit einer Innensäge wird der erfindungsgemäß ausgebildete Marknagel einschlagen, und zwar, wenn sich das Langloch in den gegenüberliegenden Abschnitten der Wand des Marknagels befindet, nur so weit, daß der um die zu verlängernde Knochenstrecke aus der Einschlagstelle vorsteht. Nach Setzen der Verriegelungsbolzen beiderseits der Osteotomie wird dann durch Längsverschiebung des Innenteils die Distraktion der Knochenenden an der Osteotomiestelle durchgeführt.

Der Hohlraum des Marknagels kann in bekannter Weise ganz oder teilweise offen sein, bevorzugt im verjüngungsseitigen Bereich, er kann aber auch völlig geschlossen sein und einen zylindrischen, polygonalen oder anders geformten Querschnitt, beispielsweise einen kleeblattförmigen Querschnitt aufweisen.

Anhand von Zeichnungen werden Ausführungsbeispiele der Erfindung näher erläutert. Es zeigen:
Fig. 1 perspektivisch eine erste Ausführungsform eines Marknagels,
Fig. 2 den Marknagel von Fig. 1 im Axialschnitt,
Fig. 3 perspektivisch eine zweite Ausführungsform des Marknagels,
Fig. 4 im Axialschnitt den Marknagel vom Fig. 3.

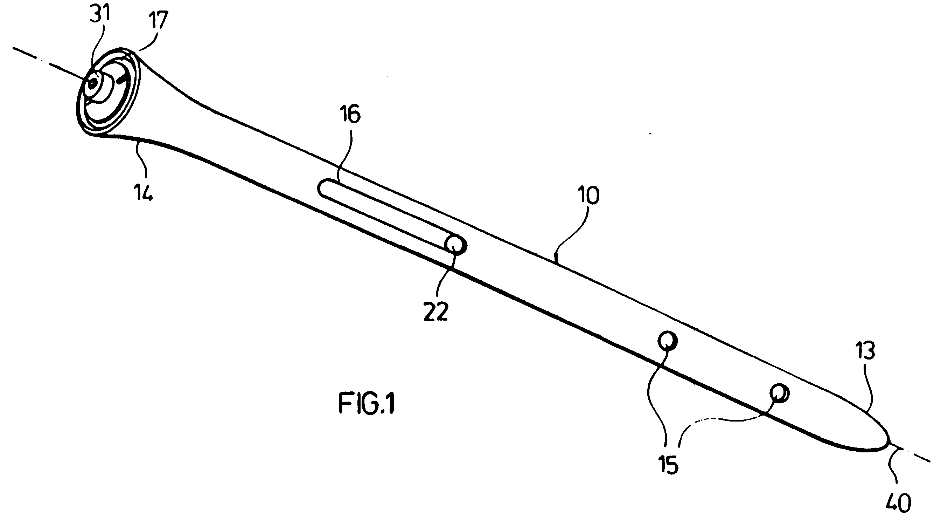

Der in den Figuren 1 bis 6 gezeigte Marknagel 10 hat eine einen zylindrischen Hohlraum 12 bildende Wand 11, deren eines sich erweiternde Eintreibende 14 mit einem Einsatz 17 versehen ist, und deren anderes Ende 13 sich zu einer Nagelspitze verjüngt. Der Einsatz 17 hat eine zentrale Bohrung 18. In der Wand 11 des Marknagels 10 sind im Bereich seines sich verjüngenden Endes 13 im Abstand angeordnete Fixierungslöcher 15 vorgesehen, die sich quer durch ihn hindurch erstrecken.

In dem zylindrischen Hohlraum 12 des Marknagels 10 ist ein zylindrisches Innenteil 20 längsverschiebbar, verdrehungsgesichert und koaxial zur Achse 40 des Marknagels 10 angeordnet. Von der dem sich erweiternden Eintreibende 14 zugewandten Stirnseite des Innenteils 20 erstreckt sich koaxial in das Innenteil 20 eine Bohrung 21 mit einem Innengewinde, in die das Außengewinde 33 einer Stange 30 eingeschraubt ist, die sich durch die Bohrung 18 im Einsatz 17 erstreckt und in einem Kopf 31 mit einem Imbusloch 32 endet. Durch Drehen des Kopfes 31 verschiebt sich das Innenteil 20 axial in dem Hohlraum 12 des Marknagels 10.

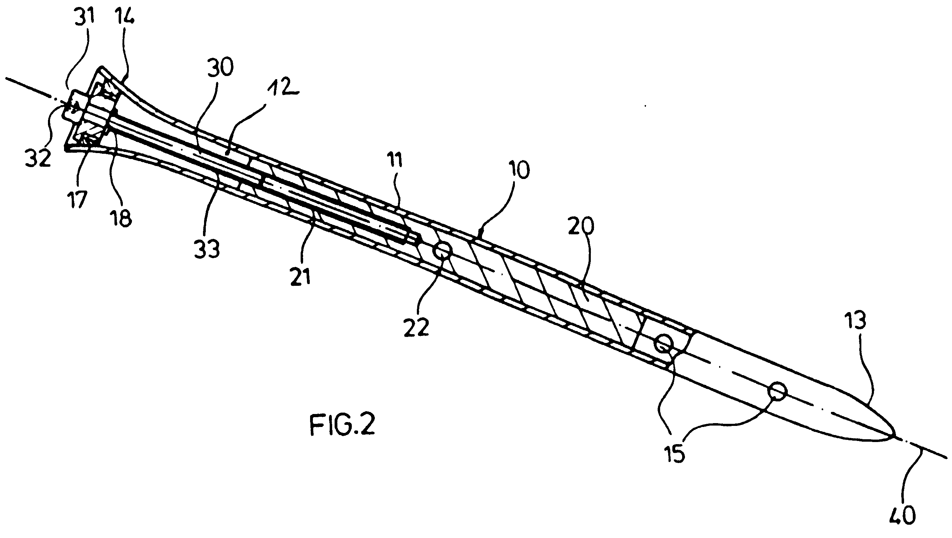

Bei der in den Figuren 1 und 2 gezeigten Ausführungsform ist im Abstand von dem Ende der Bohrung 21 mit dem Innengewinde ein sich quer durch das Innenteil 20 hindurch erstreckendes Fixierungsloch 22 ausgebildet. Fluchtend zu diesem Fixierungsloch 22 ist in der Wand 11 des Marknagels auf den gegenüberliegenden Seiten jeweils ein Langloch 16 ausgebildet. Der Marknagel 10 in der Ausführungsform der Figuren 1 und 2 wird so weit eingeschlagen, daß er proximal um die zu verlängernde Knochenstrecke aus der Einschlagstelle vorsteht. Noch Setzen der nicht gezeigten Verriegelungsbolzen durch die Fixierungslöcher 15, die sich auf der einen Seite der Osteotomie befinden, und durch die gegenüberliegenden Langlöcher 16 in der Wand 11 des Marknagels 10 und durch das Fixierungsloch 22 auf der anderen Seite der Osteotomie werden durch Drehen des Kopfes 31 der Stange 30 die Ränder der Osteotomie allmählich voneinander entfernt, wobei der zur Knochenverlängerung führende, sich erweiternde Spalt sich mit neugebildetem Knochengewebe füllt.

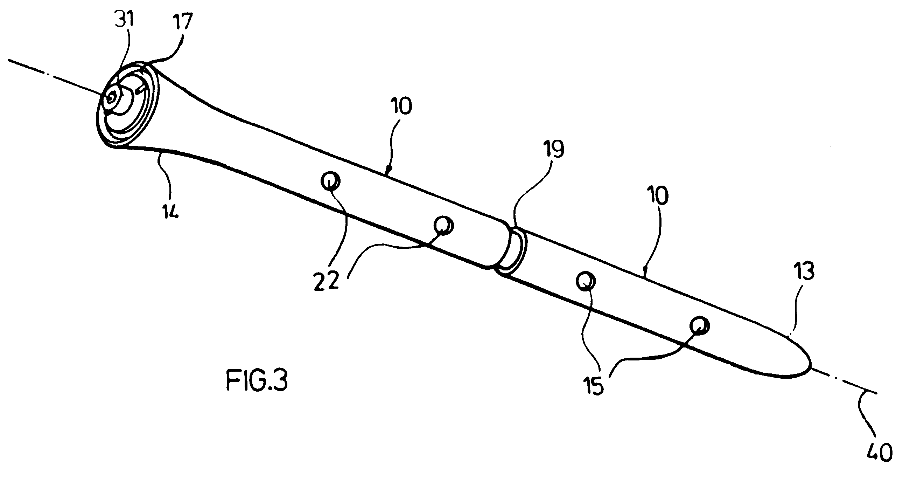

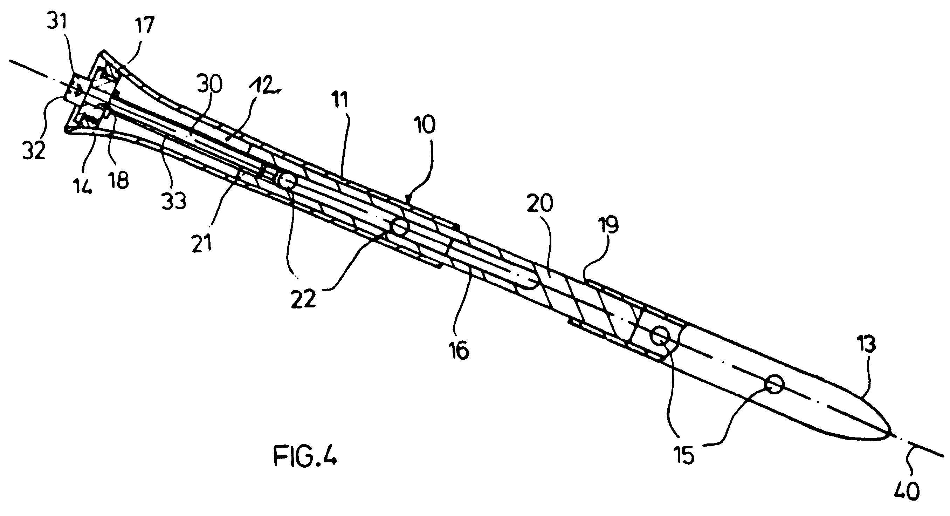

Bei der in den Figuren 3 und 4 gezeigten Ausführungsform ist die Wand 11 des Marknagels 10 an einer Trennfuge 19 durchtrennt, so daß die dadurch gebildeten beiden Teile des Marknagels 10 axial zueinander geführt auf dem Innenteil 20 verschiebbar sind. Bei dieser Ausführungsform ist das Langloch 16 in dem Innenteil 20 ausgebildet, während in der Wand 11 des eintreibseitigen Abschnitts des Marknagels 10 im Abstand in der Längserstreckung jeweils zwei fluchtende quer verlaufende Fixierungslöcher 22 auf den gegenüberliegenden Seiten fluchtend zum Langloch 16 im Innenteil 20 ausgebildet sind. Bei dieser Ausführungsform wird der Marknagel 10 vollständig in den Röhrenknochen ohne proximalen Überstand eingeschlagen. Anschließend werden die Verriegelungsbolzen gesetzt, wobei sich die Osteotomie zwischen dem Abschnitt mit dem sich verjüngenden Ende 13 und dem Abschnitt mit dem sich erweiternden Eintreibende 14 des Marknagels 10 befindet.

## Patentansprüche

1. Marknagel
- mit einer einen Hohlraum (12) bildenden Wand (11) mit einem sich verjüngenden distalen Ende (13) und einem proximalen Eintreibende (14),
- mit einem in dem Hohlraum (12) längsverschiebbar angeordneten Innenteil (20), in das sich ausgehend von seiner dem Eintreibende (14) zugewandten Stirnseite eine koaxiale Bohrung (21) mit Innengewinde erstreckt,
- mit einem im Hohlraum (12) am Eintreibende (14) vorgesehenen Einsatz (17), der eine zentrale Bohrung (18) aufweist,
- mit einer am Eintreibende (14) vorgesehenen, in der zentralen Bohrung (18) des Einsatzes (17) gelagerten Stange (30), die ein Außengewinde (33) hat, das in Eingriff mit dem Innengewinde der Bohrung (21) des Innenteils (20) steht,
- mit im Abstand in Längserstreckung der den Hohlraum (12) bildenden Wand (11) quer durchgehenden Fixierungslöchern (15),
- mit einem im Bereich des Eintreibendes (14) quer durch die den Hohlraum (12) bildende Wand (11) gehenden Langloch (16) und
- mit wenigstens einem quer durch das Innenteil (20) hindurchgehenden, fluchtend zu dem Langloch (16) in der den Hohlraum (12) bildenden Wand (11) ausgerichteten Fixierungsloch (22).

2. Marknagel
- mit einer einen Hohlraum (12) bildenden Wand (11) mit einem sich verjüngenden distalen Ende (13) und einem proximalen Eintreibende (14),
- mit einem in dem Hohlraum (12) längsverschiebbar angeordneten Innenteil (20), in das sich ausgehend von seiner dem Eintreibende (14) zugewandten Stirnseite eine koaxiale Bohrung (21) mit Innengewinde erstreckt,
- mit einem im Hohlraum (12) am Eintreibende (14) vorgesehenen Einsatz (17), der eine zentrale Bohrung (18) aufweist,
- mit einer am Eintreibende (14) vorgesehenen, in der zentralen Bohrung (18) des Einsatzes (17) gelagerten Stange (30), die ein Außengewinde (33) hat, das in Eingriff mit dem Innengewinde der Bohrung (21) des Innenteils (20) steht,
- mit im Abstand in Längserstreckung der den Hohlraum (12) bildenden Wand (11) quer durchgehenden Fixierungslöchern (15),
- mit einem im Bereich des Eintreibendes (14) quer durch das Innenteil (20) gehenden Langloch (16),
- mit wenigstens einem quer durch die den Hohlraum (12) bildende Wand (11) hindurchgehenden, fluchtend zu dem Langloch (16) in dem Innenteil (20) ausgerichteten Fixierungsloch (22) und
- mit einer Trennfuge (19) in der den Hohlraum (12) bildenden Wand (11), die die Wand (11) so trennt, daß die beiden Teile axial zueinander geführt auf dem Innenteil (20) verschiebbar sind.

3. Marknagel nach Anspruch 1 oder 2, gekennzeichnet durch einen mechanischen, pneumatischen, hydraulischen, elektrischen oder elektromagnetischen Antrieb für die Drehung der Stange (30) zur Längsverschiebung des Innenteils (20).

4. Marknagel nach Anspruch 3, dadurch gekennzeichnet, daß der Antrieb innerhalb des Hohlraums (12) angeordnet ist.

## Claims

1. Medullary nail
- having a wall (11) which forms a cavity (12) and which has a tapering distal end (13) and a proximal driving-in end (14);
- having an inner portion (20) which is arranged in a longitudinally displaceable manner in the cavity (12) and into which, starting from its end near the driving-in end (14), a coaxial bore (21) having an internal thread extends;
- having an insert (17) which is provided in the cavity (12) at the driving-in end (14) and which has a central bore (18);
- having a rod (30) which is provided at the driving-in end (14) and is mounted in the central bore (18) of the insert (17) and which has an external thread (33) which is in engagement with the internal thread of the bore (21) of the inner portion (20);
- having securing holes (15) which extend through transversely at a distance from one another in the longitudinal extent of the wall (11) forming the cavity (12);
- having a slot (16) which, in the area of the driving-in end (14), extends transversely through the wall (11) forming the cavity (12); and
- having at least one securing hole (22) which extends transversely through the inner portion (20) and is aligned with the slot (16) in the wall (11) forming the cavity (12).

2. Medullary nail
- having a wall (11) which forms a cavity (12) and which has a tapering distal end (13) and a proximal driving-in end (14);
- having an inner portion (20) which is arranged in a longitudinally displaceable manner in the cavity (12) and into which, starting from its end near the driving-in end (14), a coaxial bore (21) having an internal thread extends;
- having an insert (17) which is provided in the cavity (12) at the driving-in end (14) and which has a central bore (18);
- having a rod (30) which is provided at the driving-in end (14) and is mounted in the central bore (18) of the insert (17) and which has an external thread (33) which is in engagement with the internal thread of the bore (21) of the inner portion (20);
- having securing holes (15) which extend through transversely at a distance from one another in the longitudinal extent of the wall (11) forming the cavity (12);
- having a slot (16) which, in the area of the driving-in end (14), extends transversely through the inner portion (20);
- having at least one securing hole (22) which extends transversely through the wall (11) forming the cavity (12) and is aligned with the slot (16) in the inner portion (20); and
- having a parting line (19) in the wall (11) forming the cavity (12), which parting line (19) separates the wall (11) in such a manner that the two portions can be displaced axially relative to one another on the inner portion (20).

3. Medullary nail according to Claim 1 or 2, characterised by a mechanical, pneumatic, hydraulic, electrical or electromagnetic drive for rotating the rod (30) for the longitudinal displacement of the inner portion (20).

4. Medullary nail according to Claim 3, characterised in that the drive is arranged inside the cavity (12).

## Revendications

1. Clou médullaire comportant
- une paroi (11), formant un espace creux (12), avec une extrémité (13) distale amincie et une extrémité d'enfoncement (14) proximale,
- une partie intérieure (20) disposée de façon mobile longitudinalement dans l'espace creux (12), dans laquelle s'étend , à partir de son côté frontal orienté vers l'extrémité d'enfoncement (14), un alésage coaxial (21) à taraudage,
- un insert (17), présentant un alésage (18) central, prévu dans l'espace creux (12) à l'extrémité d'enfoncement (14),
- une tige (30) prévue à l'extrémité d'enfoncement (14), logée dans l'alésage central (18) de l'insert (17), présentant un filetage mâle (33), qui est en prise avec le taraudage de l'alésage (21) de la partie intérieure (20),
- des trous de fixation (15) transversaux disposés à intervalle dans l'allongement longitudinal de la paroi (11) formant l'espace creux (12),
- un trou oblong (16) traversant la paroi (11) formant l'espace creux (12), dans la zone de l'extrémité d'enfoncement (14) et
- au moins un trou de fixation (22) traversant transversalement la partie intérieure (20), aligné sur le trou oblong (16) et situé dans la paroi (11) formant l'espace creux (12).

2. Clou médullaire comportant
- une paroi (11), formant un espace creux (12), avec une extrémité (13) distale amincie et une extrémité d'enfoncement (14) proximale,
- une partie intérieure (20) disposée de façon mobile longitudinalement dans l'espace creux (12), dans laquelle s'étend, à partir de son côté frontal orienté vers l'extrémité d'enfoncement (14), un alésage coaxial (21) à taraudage,
- un insert (17), présentant un alésage (18) central, prévu dans l'espace creux (12) à l'extrémité d'enfoncement (14),
- une tige (30), prévue à l'extrémité d'enfoncement (14), logée dans l'alésage central (18) de l'insert (17), présentant un filetage mâle (33), qui est en prise avec le taraudage de l'alésage (21) de la partie intérieure (20),
- des trous de fixation (15) transversaux disposés à intervalle dans l'allongement longitudinal de la paroi (11) formant l'espace creux (12),
- un trou oblong (16) traversant la partie intérieure (20), dans la zone de l'extrémité d'enfoncement (14),
- au moins un trou de fixation (22) traversant transversalement la paroi (11) formant l'espace creux (12), aligné sur le trou oblong (16) de la partie intérieure (20) et
- une fente de séparation (19) située dans la paroi (11) formant l'espace creux (12), qui sépare la paroi (11) de sorte que les deux parties, guidées axialement l'une par rapport à l'autre, sont mobiles sur la partie intérieure (20).

3. Clou médullaire selon la revendication 1 ou 2, caractérisé par un mécanisme de commande mécanique, pneumatique, hydraulique, électrique ou électromagnétique pour la rotation de la tige (30) destinée au déplacement longitudinal de la partie intérieure (20).

4. Clou médullaire selon la revendication 3, caractérisé en ce que le mécanisme de commande est disposé à l'intérieur de l'espace creux (12).
